# EUROPEAN PATENT SPECIFICATION

(11) **EP 3 805 375 B1**
(45) Date of publication and mention of the grant of the patent: **18.06.2025**
(21) Application number: 18925596.1
(22) Date of filing: 03.07.2018
(51) Int. Cl.: C12N 5/10, C12N 15/867, C12N 5/071, C12N 15/65, A61K 35/12, A61P 15/08

(54) **METHOD FOR IN VITRO REPROGRAMMING OF FIBROBLASTS INTO SERTOLI CELLS AND APPLICATION THEREOF**
VERFAHREN ZUR IN-VITRO-UMPROGRAMMIERUNG VON FIBROBLASTEN IN SERTOLIZELLEN UND DEREN VERWENDUNG
PROCÉDÉ DE REPROGRAMMATION IN VITRO DE FIBROBLASTES EN CELLULES DE SERTOLI ET APPLICATION CORRESPONDANTE

(43) Date of publication of application: 14.04.2021
(73) Proprietor: Tsinghua University, Beijing 100084 (CN); Everlife Biomed Co. Ltd., Beijing 100085 (CN)
(72) Inventor: KEE, Kehkooi, Beijing 100084 (CN); LIANG, Jianlin, Beijing 100084 (CN)
(74) Representative: Murgitroyd & Company
(86) International application number: PCT/CN2018/094194
(87) International publication number: WO 2020/006675

(56) References cited:
- WO-A1-2015/147830
- WO-A2-2011/130402
- CN-A- 106 636 210
- RUIZ-BABOT GERARD ET AL: "Modeling Congenital Adrenal Hyperplasia and Testing Interventions for Adrenal Insufficiency Using Donor-Specific Reprogrammed Cells", CELL REPORTS, vol. 22, no. 5, 1 January 2018 (2018-01-01), US, pages 1236 - 1249, XP055805308, ISSN: 2211-1247, [retrieved on 20210518], DOI: 10.1016/j.celrep.2018.01.003
- DE SANTA BARBARA P ET AL: "Direct Interaction of SRY-Related Protein SOX9 and Steroidogenic Factor 1 Regulates Transcription of the Human Anti-Müllerian Hormone Gene", MOLECULAR AND CELLULAR BIOLOGY, 1 November 1998 (1998-11-01), United States, pages 6653 - 6665, XP055805407, Retrieved from the Internet <URL:https://mcb.asm.org/content/mcb/18/11/6653.full-text.pdf> [retrieved on 20210518], DOI: 10.1128/MCB.18.11.6653
- HOU YAN-PING ET AL: "Direct conversion of human fibroblasts into functional Leydig-like cells by SF-1, GATA4 and NGFI-B", AM J TRANSL RES, vol. 10, 30 January 2018 (2018-01-30), pages 175 - 183, XP055805280, Retrieved from the Internet <URL:https://www.ncbi.nlm.nih.gov/pmc/articles/PMC5801356/pdf/ajtr0010-0175.pdf> [retrieved on 20210518]
- BUGANIM, Y. ET AL.: "Direct Reprogramming of Fibroblasts into Embryonic Sertoli-Like Cells by Defined Factors", CELL STEM CELL, vol. 11, no. 3, 7 September 2012 (2012-09-07), pages 373 - 386, XP055669247
- DATABASE NUCLEOTIDE 29 April 2017 (2017-04-29), PARK, M. ET AL.: "Homo Sapiens AMH 5' Regulatory Region (LOC108783649) on Chromosome 19", XP055669249, retrieved from NCBI Database accession no. NG_051647
- KATO, T. ET AL.: "NR5Al is Required for Functional Maturation of Sertoli Cells during Postnatal Development", REPRODUCTION, vol. 143, no. 5, May 2012 (2012-05-01) - 31 December 2012 (2012-12-31), pages 663 - 672, XP055669252
- LIANG, JIANGLIN ET AL.: "Induction of Sertoli Cells from Human Fibroblasts by NR5A1 and GATA4", BIORXIV, 29 September 2018 (2018-09-29), pages 1 - 38, XP055669260
- SHIMA, Y. ET AL.: "Contribution of Leydig and Sertoli Cells to Testosterone Production in Mouse Fetal Testes", MOLECULAR ENDOCRINOLOGY, vol. 27, no. 1, 2 November 2012 (2012-11-02) - 1 January 2013 (2013-01-01), pages 63 - 73, XP055669261

## Description

### Field

The present disclosure belongs to the field of cell culture, and particularly relates to a method for in vitro reprogramming of fibroblasts into Sertoli cells and an application thereof.

### Background

According to statistics from the World Health Organization, about 15% of couples in the world are troubled by infertility, wherein a proportion of male factors is 40% to 60%, and the male factors are reflected on defects of the number or quality of sperm cells. Human pluripotent stem cells have the potential of differentiation into various cell types of human bodies. In-vitro differentiating stem cells into functional sperm cells provide a feasible approach for treatment of infertility.

Sertoli cells are one type of testis somatic cells, exist in seminiferous tubules together with germ cells, and have an important effect on triggering and regulation of spermatogenesis. For construction of a sperm in-vitro differentiation system, the Sertoli cells will be an indispensable component. In addition, the Sertoli cells in testes also evolve a special immune privilege attribute, so that the germ cells can be protected from being attacked by immune system. Such characteristic of the Sertoli cells has been applied to the allogeneic transplantation treatment of cells, tissues and organ, to relieve immune rejection from the host and improve the survival rate. In addition, the Sertoli cells per se also can be used as genetically engineered cells for carrying and expressing a particular protein or drug for treatment. Thus it can be seen that the Sertoli cells are one type of cells with great application potential; but due to restrictions of a source, ethics and the like, it is not realistic to acquire the Sertoli cells from human bodies in a large scale.

Fibroblasts are common cells in animal connective tissues, and can be obtained from animal individuals. In the cell lineage reprogramming research, the fibroblasts are often used as initiating cells; however, currently, there is no report related to in-vitro reprogramming of the fibroblasts into the Sertoli cells. Bugamin et al., Cell Stem Cell (2012) 11: 373-386 deals with embryonic Sertoli-like cells. Ruiz-Babot et al., Cell Reports (2018) 22:1236-1249 deals with steroidogenic cells and De Santa Barbara et al., Mol. Cell. Biol. (1998) 18: 6653-6665 discloses the use of a reporter system containing AMH promoter and CAT in labelling Sertoli cells.

### Summary

The present disclosure aims to prepare Sertoli cells.

The present disclosure firstly provides a method for preparing Sertoli cells, which can include a step a2): increasing the expression level and/or activity of an NR5A1 protein in fibroblasts. The invention provides a method for preparing Sertoli cells according to claims 1 to 6 and a use of a protein combination for preparing Sertoli cells according to claims 7and 8.

In the method, the "increasing the expression level and/or activity of the NR5A1 protein in the fibroblasts" can achieve an effect of increasing the expression level and/or activity of the NR5A1 protein in the fibroblasts by methods well-known in the art, such as multiple copying, change of a promoter, factor control, transgenosis and the like.

In the method, the "increasing the expression level and/or activity of the NR5A1 protein in the fibroblasts" can be introduction of a substance that increases the expression level and/or activity of the NR5A1 protein into the fibroblasts.

In the method, the "introduction of the substance that increases the expression level and/or activity of the NR5A1 protein into the fibroblasts" can be implemented by introducing nucleic acid molecules (i.e., NR5A1 genes) for encoding the NR5A1 protein into the fibroblasts. The "introducing the nucleic acid molecules for encoding the NR5A1 protein into the fibroblasts" can be implemented by infecting the fibroblasts with a lentivirus; and the lentivirus can express the NR5A1 protein.

In one embodiment of the present disclosure, the lentivirus may be a recombinant lentivirus a (expressing the NR5A1 protein). The recombinant lentivirus a can be formed by packaging of a lentivirus vector a. The lentivirus vector a, a helper plasmid vsvg and a lentivirus vector packaging plasmid pCMV ΔR8.9 can be co-transfected with 293FT cells to obtain the recombinant lentivirus a. A preparation method of the lentivirus vector a can include: inserting the NR5A1 gene to a multiple cloning site (e.g., restriction endonuclease EcoRI) of a pENTR/1A plasmid so as to obtain a vector pENTR/1A-NR5A1; inserting double-stranded DNA molecules (a nucleotide sequence of an EF1α promoter) as shown SEQ ID No. 2 in the sequence list to a multiple cloning site (e.g., the restriction endonuclease EcoRI) of a pENTR/5' topo plasmid so as to obtain a vector pENTR/5' topo-EF1α; and preparing the lentivirus vector a from the vector pENTR/1A-NR5A1, the vector pENTR/5' topo-EF1α and a lentivirus plasmid p2K7-NEO by homologous recombination.

In the method, the step a2) may include: increasing "the expression level and/or activity of the NR5A1 protein" and "the expression level and/or activity of a protein combination" in the fibroblasts; and the protein combination is at least one of a GATA4 protein, a WT1 protein, a SOX9 protein and a DMRT1 protein.

In the method, the "increasing 'the expression quantity and/or level of the NR5A1 protein' and 'the expression level and/or activity of the protein combination' in the fibroblasts" can achieve an effect of increasing "the expression level and/or activity of the NR5A1 protein" and "the expression level and/or activity of the protein combination" in the fibroblasts by methods well-known in the art, such as multiple copying, change of the promoter, factor control, transgenosis and the like.

In the method, the "increasing 'the expression level and/or activity of the NR5A1 protein' and 'the expression level and/or activity of the protein combination' in the fibroblasts" can be introduction of "the substance that increases the expression level and/or activity of the NR5A1 protein" and "a substance that increases the expression level and/or activity of the protein combination" into the fibroblasts.

The "introduction of 'the substance that increases the expression level and/or activity of the NR5A1 protein' and 'the substance that increases the expression level and/or activity of the protein combination' into the fibroblasts" can be implemented by introducing the nucleic acid molecules (i.e., the NR5A1 genes) for encoding the NR5A1 protein and nucleic acid molecules for encoding the protein combination into the fibroblasts. Nucleic acid molecules for encoding the GATA4 protein namely are GATA4 genes. Nucleic acid molecules for encoding the WT1 protein namely are WT1 genes. Nucleic acid molecules for encoding the SOX9 protein namely are SOX9 genes. Nucleic acid molecules for encoding the DMRT1 protein namely are DMRT1 genes. The "introducing the nucleic acid molecules for encoding the NR5A1 protein and the nucleic acid molecules for encoding the protein combination into the fibroblasts" can be implemented by infecting the fibroblasts with a lentivirus; and the lentivirus can express the NR5A1 protein and the protein combination.

In one embodiment of the present disclosure, the lentivirus expressing the NR5A1 protein may be the recombinant lentivirus a.

In one embodiment of the present disclosure, the lentivirus expressing the GATA4 protein may be a recombinant lentivirus b. The recombinant lentivirus b can be formed by packaging of a lentivirus vector b. The lentivirus vector b, the helper plasmid sag and the lentivirus vector packaging plasmid pCMV ΔR8.9 can be co-transfected with the 293FT cells to obtain the recombinant lentivirus b. A preparation method of the lentivirus vector b can include: inserting the GATA4 gene to the multiple cloning site (e.g., the restriction endonuclease EcoRI) of the pENTR/1A plasmid so as to obtain a vector pENTR/1A-GATA4; and preparing the lentivirus vector b from the vector pENTR/1A-GATA1, the vector pENTR/5' topo-EF1α and the lentivirus plasmid p2K7-NEO by homologous recombination.

In one embodiment of the present disclosure, the lentivirus expressing the WT1 protein may be a recombinant lentivirus c. The recombinant lentivirus c can be formed by packaging of a lentivirus vector c. The lentivirus vector c, the helper plasmid vsvg and the lentivirus vector packaging plasmid pCMV ΔR8.9 can be co-transfected with the 293FT cells to obtain the recombinant lentivirus c. A preparation method of the lentivirus vector c can include: inserting the WT1 gene to the multiple cloning site (e.g., between restriction endonucleases NotI and AscI) of the pENTR/D-topo plasmid so as to obtain a vector pENTR/D-toppo-WT1; and preparing the lentivirus vector c from the vector pENTR/D-topo-WT1, the vector pENTR/5' topo-EF1α and a lentivirus plasmid 2K7-BSD by homologous recombination.

In one embodiment of the present disclosure, the lentivirus expressing the SOX9 protein may be a recombinant lentivirus d. The recombinant lentivirus d can be formed by packaging of a lentivirus vector d. The lentivirus vector d, the helper plasmid vsvg and the lentivirus vector packaging plasmid pCMV ΔR8.9 can be co-transfected with the 293FT cells to obtain the recombinant lentivirus d. A preparation method of the lentivirus vector d can include: inserting the SOX9 gene to the multiple cloning site (e.g., between the restriction endonucleases NotI and AscI) of the pENTR/D-topo plasmid so as to obtain a vector pENTR/D-topo-SOX9; and preparing the lentivirus vector d from the vector pENTR/D-topo-SOX9, the vector pENTR/5' topo-EF1α and the lentivirus plasmid p2K7-NEO by homologous recombination.

In one embodiment of the present disclosure, the lentivirus expressing the DMRT1 protein may be a recombinant lentivirus e. The recombinant lentivirus e can be formed by packaging of a lentivirus vector e. The lentivirus vector e, the helper plasmid vsvg and the lentivirus vector packaging plasmid pCMV ΔR8.9 can be co-transfected with the 293FT cells to obtain the recombinant lentivirus e. A preparation method of the lentivirus vector e can include: inserting the DMRT1 gene to the multiple cloning site (e.g., the restriction endonuclease EcoRI) of the pENTR/1A plasmid so as to obtain a vector pENTR/1A-DMRT1; and preparing the lentivirus vector e from the vector pENTR/1A-DMRT1, the vector pENTR/5' topo-EF1α and the lentivirus plasmid p2K7-NEO by homologous recombination.

Any one of the "introducing the nucleic acid molecules for encoding the NR5A1 protein into the fibroblasts" or any one of the "introducing the nucleic acid molecules for encoding the NR5A1 protein and the nucleic acid molecules for encoding the protein combination into the fibroblasts" can be implemented by adopting the corresponding lentivirus infection. Time of the infection may be 6 to 28h (e.g., 6 to 20h, 20 to 24h, 24 to 28h, 6h, 20h, 24h or 28h). After the infection is finished, a medium for culturing the fibroblasts needs to be added, and culture is carried out for 20 to 28h (e.g., 20 to 24h, 24 to 28h, 20h, 24h or 28h) under the conditions of a temperature of 35 to 39°C (e.g., 35 to 37°C, 37 to 39°C, 35°C, 37°C or 39°C) and 5% CO₂.

Any one of the above-mentioned methods may further include a step a1): introducing a fluorescent protein reporter system specifically expressed in the Sertoli cells into the fibroblasts; in the fluorescent protein reporter system specifically expressed in the Sertoli cells, a promoter specifically expressed in the Sertoli cells promotes expression of a fluorescent protein; and the step a1) is carried out before the step a2) is carried out.

In the fluorescent protein reporter system, the promoter specifically expressed in the Sertoli cells may be a promoter of an AMH gene. A nucleotide sequence of the promoter of the AMH gene can be as shown in SEQ ID No. 1 in the sequence list.

In the fluorescent protein reporter system, the fluorescent protein is a green fluorescent protein, a yellow fluorescent protein or a red fluorescent protein.

Any one of the above-mentioned mediums for culturing the fibroblasts may be a DMEM medium containing 8 to 12% (e.g., 8 to 10%, 10 to 12%, 8%, 10% or 12%) (v/v) of FBS, 0.7 to 1.3% (e.g., 0.7 to 1.0%, 1.0 to 1.3%, 0.7%, 1.0% or 1.3%) (m/v) of glutamic acid and 1% (e.g., 0.7 to 1.0%, 1.0 to 1.3%, 0.7%, 1.0% or 1.3%) (m/v) of non-essential amino acid.

Any one of the above-mentioned methods can further include steps a3) to a6):
a3): After completing the step a2), adding G418 into a culture system of the fibroblasts to obtain a medicinal sieve system;
a4): After completing the step a3), taking the medicinal sieve system and culturing for 3-7d (e.g., 3-5d, 5-7d, 3d, 5d or 7d);
a5): After completing the step a4), discarding the liquid phase, adding the medium for culturing the fibroblasts and continuing culturing for 3-7d (e.g., 3-5d, 5-7d, 3d, 5d or 7d); and
a6): after completing the step a5), isolating the Sertoli cells from the culture system.

In the step a3), concentration of the G418 may be 0.7 to 1.3mg/mL (e.g., 0.7 to 1.0mg/mL, 1.0 to 1.3mg/mL, 0.7mg/mL, 1.0mg/mL or 1.3mg/mL).

In the step a5), the medium for culturing the fibroblasts may also be a DMEM/F12 medium containing 8 to 12% (e.g., 8 to 10%, 10 to 12%, 8%, 10% or 12%) (v/v) of FBS.

In the step a6), the isolating the Sertoli cells can be implemented by isolating cells capable of emitting fluorescence.

Any one of the above-mentioned cultures can be carried out at a temperature of 35 to 39°C (e.g., 35 to 37°C, 37 to 39°C, 35°C, 37°C or 39°C).

Any one of the above-mentioned fluorescent protein reporter systems specifically expressed in the Sertoli cells also falls within the protection scope of the present disclosure.

The present disclosure further provides an application of any one of the above-mentioned fluorescent protein reporter systems specifically expressed in the Sertoli cells, which may be at least one of c1) to c4):
c1) Application as an indication mark of the Sertoli cells;
c2) Application as a separation mark of the Sertoli cells;
c3) Application as an enrichment mark of the Sertoli cells; and
c4) Application to preparation of the Sertoli cells.

The present disclosure further provides S1) or S2):
S1): Application of an NR5A1 protein to preparation of Sertoli cells; and
S2): Application of the NR5A1 protein and a protein combination to preparation of the Sertoli cells, the protein combination being at least one of a GATA4 protein, a WT1 protein, a SOX9 protein and a DMRT1 protein.

The present disclosure further provides T1) or T2):
T1) Application of nucleic acid molecules for encoding a NR5A1 protein to preparation of Sertoli cells; and
T2) Application of the nucleic acid molecules for encoding the NR5A1 protein and nucleic acid molecules for encoding a protein combination to preparation of the Sertoli cells, the protein combination being at least one of a GATA4 protein, a WT1 protein, a SOX9 protein and a DMRT1 protein.

The present disclosure further provides an application of fibroblasts to preparation of Sertoli cells.

The present disclosure further provides a product for preparing Sertoli cells; and the product may contain a NR5A1 protein.

The product may further contain a protein combination; and the protein combination may be at least one of a GATA4 protein, a WT1 protein, a SOX9 protein and a DMRT1 protein.

The product may further contain fibroblasts.

The product may further contain any one of the above-mentioned fluorescent protein reporter systems specifically expressed in the Sertoli cells.

The product may further contain fibroblasts introduced into the fluorescent protein reporter system specifically expressed in the Sertoli cells.

Any one of the above-mentioned products may further contain G418 and/or the medium for culturing the fibroblasts and/or the DMEM/F12 medium containing 8 to 12% (e.g., 8 to 10%, 10 to 12%, 8%, 10% or 12%) (v/v) of FBS.

The present disclosure further provides specific DNA molecules as shown in the SEQ ID No. 1 in the sequence list.

Application of the specific DNA molecules to promotion of target gene expression also falls within the protection scope of the present disclosure.

In the application, the target gene expression may be expression of a target gene in Sertoli cells.

The present disclosure further provides a method for expressing a target gene, including the following step of: inserting the specific DNA molecules to the upstream of any target gene so as to promote target gene expression.

In the method, the target gene expression may be expression of the target gene in Sertoli cells.

Any one of the above-mentioned fibroblasts may be human fibroblasts. The human fibroblasts may be dH1 fibroblast strains or human lung fibroblast strains.

A preparation method of the dH1 fibroblast strains may be as follows:
(1) Transferring human embryonic stem cells to a culture plate coated by a 1% Matrigel stock solution, adding an ES medium, and culturing in a 5% CO₂ thermostatic incubator with a temperature of 37°C for 5d.the ES medium may be a Knockout DMEM medium containing 20% (v/v) of KSR, 1% (m/v) of glutamic acid, 1% (m/v) of non-essential amino acid and 4ng/mL of bFGF; and
(2) After completing the step (1), discarding the medium, adding a differentiation medium, and continuing culturing in the 5% CO₂ thermostatic incubator with the temperature of 37°C for 7 to 10d until the embryonic stem cells are differentiated into the fibroblasts, so as to obtain the dH1 fibroblast strains. the differentiation medium maybe a Knockout DMEM medium containing 10% (v/v) of FBS, 1% (m/v) of glutamic acid and 1% (m/v) of non-essential amino acid.

Any one of the above-mentioned lentivirus plasmids p2K7-NEO, any one of the above-mentioned pENTR/1A plasmids and any one of the above-mentioned pENTR/5' topo plasmids all can be products of Invitrogen Corporation. Any one of the above-mentioned DMEM/F12 mediums can be a product of Corning Inc.

Any one of the above-mentioned helper plasmids vsvg and any one of the above-mentioned lentivirus vector packaging plasmids pCMV ΔR8.9 both can be products of Invitrogen Corporation. Any one of the above-mentioned non-essential amino acids can be a product of Gibco Corporation.

The Gene ID of any one of the above-mentioned NR5A1 proteins is 2516. The Refseq number of any one of the above-mentioned NR5A1 genes is NC_000009.12. The Gene ID of any one of the above-mentioned GATA4 proteins is 2626. The Refseq number of any one of the above-mentioned GATA4 genes is NC_000008.11. The Gene ID of any one of the above-mentioned WT1 proteins is 7409. The Refseq number of any one of the above-mentioned WT1 genes is NC_000011.10. The Gene ID of any one of the above-mentioned SOX9 proteins is 6662. The Refseq number of any one of the above-mentioned SOX9 genes is NC_000017.11. The Gene ID of any one of the above-mentioned DMRT1 proteins is 1761. The Refseq number of any one of the above-mentioned DMRT1 genes is NC_000009.12.

Any one of the above-mentioned green fluorescent proteins is a fluorescent protein eGFP (the Gene ID is 20473140). The Refseq number of a gene (an eGFP gene) for encoding the fluorescent protein eGFP is NC_025025.1.

Experiments prove that the hiSC prepared by adopting the method provided by the present disclosure highly expresses KRT18 and presents an epithelial appearance, its gene is enriched in the important biological process of the Sertoli cells, and the hiSC has the characteristics of attracting endothelial cells, forming lipid droplets, interacting with germ cells, inhibiting lymphocyte growth and interleukin production, having immune privilege and the like, and fully acquires the characteristics of the Sertoli cells. The present disclosure provides a rich cell source for constructing a complete in-vitro sperm differentiation system and researching an interaction mechanism between the Sertoli cells and male germ cells. The present disclosure has important application value in the fields of infertility treatment, allogeneic transplantation, cell therapy and the like.

### Brief Description of the Drawings

Fig. 1 is a location diagram of an AMH: GFP reporter system and blasticidin (BSD) in a recombinant lentivirus.
Fig. 2 is a flow schematic diagram of in vitro reprogramming of human fibroblasts into hiSCs.
Fig. 3 is an analysis result of flow cytometry of a step I in an embodiment 2.
Fig. 4 is a transcriptome map of part of to-be-detected cells.
Fig. 5 is GO analysis of the transcriptome map.
Fig. 6 is detection of an expression level of KRT18 in 2F-hiSCs (dH1) by cell immunofluorescence staining.
Fig. 7 is detection on an ability that the 2F-hiSCs (dH1) attract human umbilical vein endothelial cells (HUVECs).
Fig. 8 is detection on an ability that the 2F-hiSCs (dH1) form lipid droplets.
Fig. 9 is detection on an ability that the 2F-hiSCs (dH1) interact with germ cells.
Fig. 10 shows that the 2F-hiSCs (dH1) can inhibit lymphocyte growth and interleukin production.
Fig. 11 is detection on immune privilege of the 2F-hiSCs (dH1).

### Detailed Description of the Embodiments

The present disclosure will be further described in detail below in combination of particular embodiments, and given embodiments are merely used for illustrating the present disclosure, but not intended to limit the scope of the present disclosure.

Experimental methods in the under-mentioned embodiments are all conventional methods, unless otherwise specified.

Materials, reagents and the like used in the under-mentioned embodiments all can be obtained commercially, unless otherwise specified.

For quantitative tests of the following embodiments, repeated experiments are set for three times, and results are averaged.

A lentivirus plasmid p2K7-NEO, a pENTR/1A plasmid and a pENTR/5' topo plasmid are all products of Invitrogen Corporation. A human lung fibroblast strain is a product of Concord Cell Bank. Both a helper plasmid vsvg and a lentivirus vector packaging plasmid pCMV ΔR8.9 are products of Invitrogen Corporation. Both WST-1 solution and RIPA solution are products of Beyotime Corporation. Both Matrigel stock solution and a DMEM/F12 medium are products of Corning Inc. Both bFGF and Polybrene are products of R&D Corporation. Non-essential amino acid is a product of Gibco Corporation. HUVECs are products of ATCC Corporation. A Transwell (a pore diameter is 8µm) is a product of Corning Inc. an ECM medium (i.e., a migration medium) is a product of ScienCell Corporation. A BODIPY dye is a product of Thermofishe Corporation. An ELASA kit is a product of Cusabio Corporation.

An ES medium is a Knockout DMEM medium containing 20% (v/v) of KSR, 1% (m/v) of glutamic acid, 1% (m/v) of non-essential amino acid and 4ng/mL of bFGF.

A differentiation medium is a Knockout DMEM medium containing 10% (v/v) of FBS, 1% (m/v) of glutamic acid and 1% (m/v) of non-essential amino acid.

An MEF medium is a DMEM medium containing 10% (v/v) of FBS, 1% (m/v) of glutamic acid and 1% (m/v) of non-essential amino acid. The MEF medium is used for culturing MEF.

Preparation of a dH1 fibroblast strain includes the steps as follows:
(1) Transferring human embryonic stem cells to a culture plate coated by a 1% Matrigel stock solution, adding the ES medium, and culturing in a 5% CO₂ thermostatic incubator with a temperature of 37°C for 5d; and
(2) After completing the step (1), discarding the medium, adding the differentiation medium, and continuing culturing in the 5% CO₂ thermostatic incubator with the temperature of 37°C for 7 to 10d until the embryonic stem cells are differentiated into the fibroblasts, so as to obtain the dH1 fibroblast strain.

The Gene ID of an NR5A1 protein is 2516. The Refseq number of an NR5A1 gene is NC_000009.12.

The Gene ID of a GATA4 protein is 2626. The Refseq number of a GATA4 gene is NC_000008.11.

The Gene ID of a WT1 protein is 7409. The Refseq number of a WT1 gene is NC_000011.10.

The Gene ID of an SOX9 protein is 6662. The Refseq number of an SOX9 gene is NC_000017.11.

The Gene ID of a DMRT1 protein is 1761. The Refseq number of a DMRT1 gene is NC_000009.12.

The Gene ID of a fluorescent protein eGFP is 20473140. The Refseq number of an eGFP gene is NC_025025.1.

### Embodiment 1: Construction of Fibroblast Strain Containing AMH:GFP Reporter System

A fibroblast strain is a dH1 fibroblast strain or a human lung fibroblast strain.

### Step I: construction of the AMH:GFP reporter system

1. An inventor of the present disclosure clones a first promoter of an AMH gene from human genome DNA. A length of the first promoter of the AMH gene is about 1.6kb, and a nucleotide sequence of the first promoter is as shown in a SEQ ID No. 1 in the sequence list.
2. After the step 1 is completed, an encoding gene (i.e., an eGFP gene) of a fluorescent protein eGFP is connected to a 3' terminal of the first promoter of the AMH gene so as to obtain the AMH: GFP reporter system. In the AMH: GFP reporter system, the first promoter of the AMH gene drives expression of the fluorescent protein eGFP.

AMH (Gene ID: 268) is a marker protein specifically expressed in the early period of development of Sertoli cells. If the Sertoli cells are formed in cells introduced into the AMH: GFP reporter system, green fluorescence will be emitted.

### Step II: construction of a recombinant lentivirus containing the AMH: GFP reporter system

1. The eGFP gene is inserted to a recognition site of restriction endonuclease EcoRI of a pENTR/1A plasmid so as to obtain a vector pENTR/1A-eGFP.
2. Double-stranded DNA molecules shown in the SEQ ID No. 1 in the sequence list are inserted to a recognition site of restriction endonuclease EcoRI of a pENTR/5' topo plasmid so as to obtain a vector pENTR/5' topo-AMH.
3. The vector pENTR/1A-eGFP, the vector pENTR/5' topo-AMH and a lentivirus plasmid p2K7-NEO are taken to prepare a lentivirus vector by homologous recombination.
4. The lentivirus vector prepared in the step 3, a helper plasmid vsvg and a lentivirus vector packaging plasmid pCMV ΔR8.9 are co-transfected with 293FT cells to obtain the recombinant lentivirus containing the AMH: GFP reporter system.

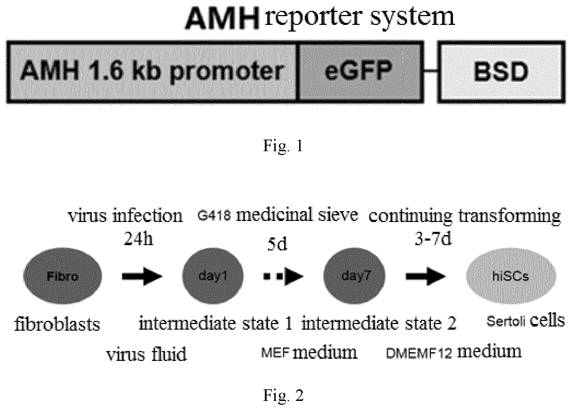

In the recombinant lentivirus containing the AMH: GFP reporter system, a location diagram of the AMH: GFP reporter system and blasticidin (BSD) refers to Fig. 1.

### Step III: construction of a fibroblast strain containing the AMH:GFP reporter system

By a lentivirus infection manner, the recombinant lentivirus containing the AMH: GFP reporter system obtained in the step II is integrated to a fibroblast strain (the BSD carried by the recombinant lentivirus containing the AMH: GFP reporter system is used as a selection marker), so as to obtain the fibroblast strain containing the AMH: GFP reporter system.

Hereinafter, the dH1 fibroblast strain containing the AMH: GFP reporter system is referred to as dH1•AMH: GFP for short. An HPF fibroblast strain containing the AMH: GFP reporter system is referred to as HPF•AMH: GFP for short.

### Embodiment 2: In vitro Reprogramming of Human Fibroblasts into AMH:GFP+Sertoli cells (hereinafter referred to as hiSCs for short)

A flow schematic diagram of in vitro reprogramming of the human fibroblasts into the hiSCs refers to Fig. 2.

### Step I: in vitro reprogramming of a dH1 fibroblast strain into the hiSCs (dH1)

### 1. A recombinant lentivirus is prepared.

(1) The NR5A1 gene is inserted to a recognition site of the restriction endonuclease EcoRI of the pENTR/1A plasmid so as to obtain a vector pENTR/1A-NR5A1. The GATA4 gene is inserted to the recognition site of the restriction endonuclease EcoRI of the pENTR/1A plasmid so as to obtain a vector pENTR/1A-GATA4. The WT1 gene is inserted between recognition sites of restriction endonucleases NotI and AscI of a pENTR/D-topo plasmid so as to obtain a vector pENTR/D-topo-WT1. The SOX9 gene is inserted between recognition sites of the restriction endonucleases NotI and AscI of the pENTR/D-topo plasmid so as to obtain a vector pENTR/D-topo-SOX9. The DMRT1 gene is inserted to a recognition site of the restriction endonuclease EcoRI of the pENTR/1A plasmid so as to obtain a vector pENTR/1A-DNMT1.
(2) Double-stranded DNA molecules (a nucleotide sequence of an EF1α promoter) shown in a SEQ ID No. 2 in the sequence list are inserted to a recognition site of restriction endonuclease EcoRI of a pENTR/5' topo plasmid so as to obtain a vector pENTR/5' topo-EF1α.
(3) The vector pENTR/1A-NR5A1, the vector pENTR/5' topo-EF1α and the lentivirus plasmid p2K7-NEO are taken to prepare a lentivirus vector a by homologous recombination. The lentivirus vector a, the helper plasmid vsvg and the lentivirus vector packaging plasmid pCMV ΔR8.9 are co-transfected with 293FT cells to obtain the recombinant lentivirus a.

According to the method in the step (3), the vector pENTR/1A-NR5A1 is respectively replaced with the vector pENTR/1A-GATA4, the vector pENTR/1A-WT1, the vector pENTR/1A-SOX9 and the vector pENTR/1A-DMRT1, and other steps are unchanged, so as to sequentially obtain a recombinant lentivirus b, a recombinant lentivirus c, a recombinant lentivirus d and a recombinant lentivirus e.

The recombinant lentivirus an express the NR5A1 protein. The recombinant lentivirus b expresses the GATA4 protein. The recombinant lentivirus c expresses the WT1 protein. The recombinant lentivirus d expresses the SOX9 protein. The recombinant lentivirus e expresses the DMRT1 protein.

2. A T175 culture flask is inoculated with the dH1•AMH: GFP (an inoculation density is 30% to 60%), and then 1mL of virus fluid (protein combinations expressed by the recombinant lentiviruses in the virus fluid respectively refer to No.1 to No.31 in Table 1, an adding amount of each type of viruses is 200µL, and if a total volume is less than 1mL, supplementation is carried out by the MEF medium) containing the recombinant lentiviruses and Polybrene is added to obtain an infection system. In the infection system, a density of the dH1•AMH: GFP is 40 to 70%, and concentration of the Polybrene is 8ng/mL.

The T175 culture flask is inoculated with the dH1•AMH: GFP (the inoculation density is 30% to 60%), and then 1mL of MEF medium containing the Polybrene is added to obtain an infection system (as a control, i.e., No.0 in Table 1). In the infection system, a density of the dH1•AMH: GFP is 40 to 70%, and concentration of the Polybrene is 8ng/mL. Combinations NG, NGD, WNG, SNG, WNGD, SNGD and SWNG are according to the invention. The other combinations are not according to the invention.

**Table 1**

| Nu mbe r | Abbreviation of Protein Combination Type | Protein Combinations | | | | |
|---|---|---|---|---|---|---|
| | | NR5A1 Protein | GATA4 Protein | SOX9 Protein | WT1 Protein | DMRTI1 Protein |
| 1 | S | - | - | + | - | - |
| 2 | W | - | - | - | + | - |
| 3 | N | + | - | - | - | - |
| 4 | G | - | + | - | - | - |
| 5 | D | - | - | - | - | + |
| 6 | SW | - | - | + | + | - |
| 7 | SN | + | - | + | - | - |
| 8 | SG | - | + | + | - | - |
| 9 | SD | - | - | + | - | + |
| 10 | WN | + | - | - | + | - |
| 11 | WG | - | + | - | + | - |
| 12 | WD | - | - | - | + | + |
| 13 | NG | + | + | - | - | - |
| 14 | ND | + | - | - | - | + |
| 15 | GD | - | + | - | - | + |
| 16 | NGD | + | + | - | - | + |
| 17 | WGD | - | + | - | + | + |
| 18 | WND | + | - | - | + | + |
| 19 | WNG | + | + | - | + | - |
| 20 | SGD | - | + | + | - | + |
| 21 | SND | + | - | + | - | + |
| 22 | SNG | + | + | + | - | - |
| 23 | SWD | - | - | + | + | + |
| 24 | SWG | - | + | + | + | - |
| 25 | SWN | + | - | + | + | - |
| 26 | WNGD | + | + | - | + | + |
| 27 | SNGD | + | + | + | - | + |
| 28 | SWGD | - | + | + | + | + |
| 29 | SWND | + | - | + | + | + |
| 30 | SWNG | + | + | + | + | - |
| 31 | SWNGD | + | + | + | + | + |
| 0 | CTRL | - | - | - | - | - |

| | | | | | | |
|---|---|---|---|---|---|---|
| Notes: + represents that the protein combination contains a certain protein, and - represents that the protein combination does not contain a certain protein. | | | | | | |

3. After the step 2 is completed, the infection systems are respectively taken and placed in the 5% CO₂ thermostatic incubator with the temperature of 37°C to stand for 24h.
4. The infection systems subjected to the step 3 are respectively taken, a liquid phase is discarded, the MEF medium is added, and the obtained product is placed into the 5% CO₂ thermostatic incubator with the temperature of 37°C to stand for 24h (an intermediate state 1 in Fig. 2).
5. After the step 4 is completed, G418 is respectively added to obtain a system 1; and in the system 1, concentration of the G418 is 1mg/mL.
6. After the step 5 is completed, the system 1 is respectively taken and placed into the 5% CO₂ thermostatic incubator with the temperature of 37 °C to be cultured for 5d (an intermediate state 2 in Fig. 2), wherein when culture is carried out on the third to fourth day, the medium needs to be replaced with an MEF culture medium containing 1mg/mL of G418.
7. The system 1 subjected to the step 6 is respectively taken, the medium is discarded, and a DMEM/F12 medium containing 10% (v/v) of FBS is added to obtain a system 2.
8. After the step 7 is completed, the system 2 is respectively taken and placed into the 5% CO₂ thermostatic incubator with the temperature of 37°C to be subjected to static culture for 3 to 7d.
9. The system 2 subjected to the step 8 is respectively taken, and analysis is carried out by adopting flow cytometry.

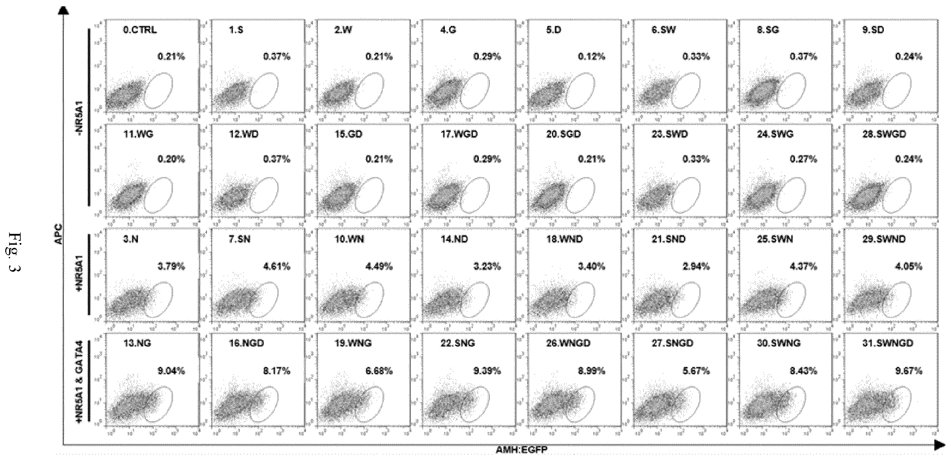

A result of the flow cytometry refers to Fig. 3. The result shows that when the protein combination expressed by the recombinant lentivirus in the virus fluid in the step 2 is No.3, No.7, No.10, No.13, No.14, No.16, No.18, No.19, No.21, No.22, No.25, No.26, No.27, No.29, No.30 or No.31, the AMH: GFP+Sertoli cells (hereinafter referred to as hiSCs (dH1) for short) can be obtained.

10. The system 2 subjected to the step 8 is taken, and cells emitting green fluorescence, i.e., the hiSCs (dH1), are collected.

AMH: GFP+Sertoli cells obtained by the NR5A1 protein and the GATA4 protein (No.13in Table 1) expressed by the recombinant lentivirus in the virus fluid are referred to as 2F-hiSCs (dH1) for short.

AMH: GFP+Sertoli cells obtained by the NR5A1 protein, the GATA4 protein, the WT1 protein, the SOX9 protein and the DMRT1 protein (No.31 in Table 1) expressed by the recombinant lentivirus in the virus fluid are referred to as 5F-hiSCs (dH1) for short.

According to the steps 2 to 9, 1mL of virus fluid containing the recombinant lentivirus and the Polybrene is replaced with 1mL of virus fluid containing an empty vector lentivirus (obtained by co-transfecting the lentivirus plasmid p2K7-NEO, the helper plasmid vsvg and the lentivirus vector packaging plasmid pCMVΔR8.9 with the 293FT cells) and the Polybrene (a volume of the empty vector lentivirus is 200µL, and a total volume is supplemented to 1mL with the MEF medium), and other steps are all unchanged. The result of the flow cytometry shows that in the system 2 subjected to the step 8, cells are dH1-2K7 cells (hereinafter referred to as dH1-2K7 for short), and the hiSCs (dH1) are nearly not obtained.

According to the steps 2 to 9, the dH1•AMH: GFP is replaced with the dH1 fibroblast strain, and other steps are all unchanged. The result of the flow cytometry shows that in the system 2 subjected to the step 8, the hiSCs (dH1) are nearly not obtained.

### Step II: in vitro reprogramming of a human lung fibroblast strain into the hiSCs (HPF)

According to the step I, the dH1 fibroblast strain is replaced with the human lung fibroblast strain, and other steps are all unchanged. A result shows that when the protein combination expressed by the recombinant lentivirus in the virus fluid in the step 2 is No.3, No.7, No.10, No.13, No.14, No.16, No.18, No.19, No.21, No.22, No.25, No.26, No.27, No.29, No.30 or No.31, the AMH: GFP+Sertoli cells (hereinafter referred to as hiSCs (HPF) for short) can be obtained. AMH: GFP+Sertoli cells obtained by the NR5A1 protein and the GATA4 protein (No.13 in Table 1) expressed by the recombinant lentivirus in the virus fluid are referred to as 2F-hiSCs (HPF) for short. AMH: GFP+Sertoli cells obtained by the NR5A1 protein, the GATA4 protein, the WT1 protein, the SOX9 protein and the DMRT1 protein (No.31 in Table 1) expressed by the recombinant lentivirus in the virus fluid are referred to as 5F-hiSCs (HPF) for short.

According to the steps 2 to 9 in the step I, 1mL of virus fluid containing the recombinant lentivirus and the Polybrene is replaced with 1mL of virus fluid containing an empty vector lentivirus (obtained by co-transfecting the lentivirus plasmid p2K7-NEO, the helper plasmid vsvg and the lentivirus vector packaging plasmid pCMVΔR8.9 with the 293FT cells) and the Polybrene (a volume of the empty vector lentivirus is 200µL, and a total volume is supplemented to 1mL with the MEF medium), and dH1•AMH:GFP is replaced with HPF•AMH:GFP, and other steps are all unchanged. The result of the flow cytometry shows that in the system 2 subjected to the step 8, cells are HPF-2K7 cells (hereinafter referred to as HPF-2K7 for short), and the hiSCs (HPF) are nearly not obtained.

### Embodiment 3: Characteristics of hiSCs obtained by In vitro Reprogramming of Human fibroblasts in Embodiment 2

### Step I: analysis of a transcriptome map

The transcriptome map of to-be-detected cells (2F-hiSCs (dH1), 5F-hiSCs (dH1), 2F-hiSCs (HPF), 5F-hiSCs (HPF), adult Sertoli cells or dH1-2K7) is compared by adopting an RNA sequencing method, and then GO analysis is carried out.

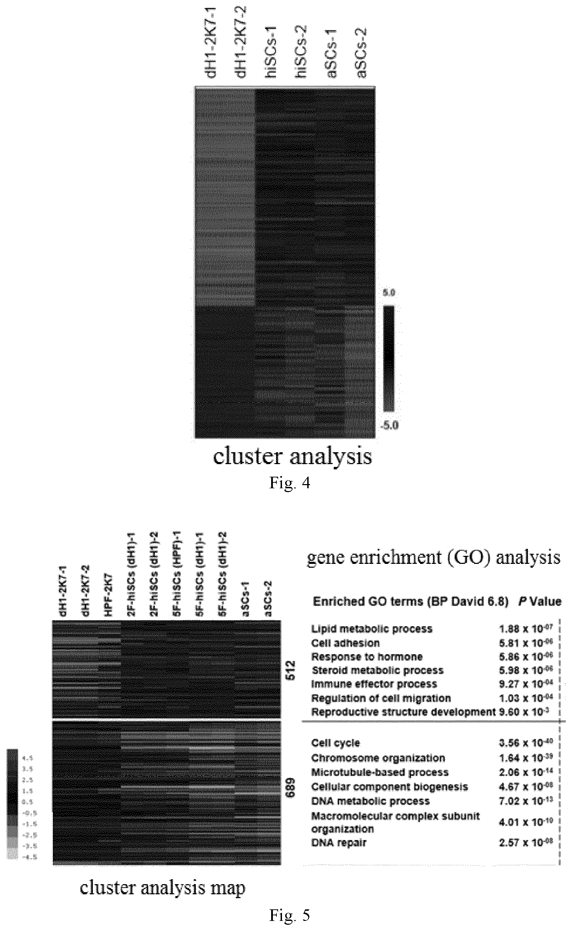

Part of the transcriptome map refers to Fig. 4 (both dH1-2K7-1 and dH1-2K7-2 are dH1-2K7 and used as controls; both hiSCs-1 and hiSCs-2 are 5F-hiSCs (dH1), and both aSCs-1 and aSCs-2 are the adult Sertoli cells).

Part of the GO analysis result refers to Fig. 5 (both dH1-2K7-1 and dH1-2K7-2 are dH1-2K7 and used as controls; both 2F-hiSCs (dH1)-1 and 2F-hiSCs (dH1)-2 are 2F-hiSCs (dH1), 5F-hiSCs (HPF)-1 is 5F-hiSCs (HPF), both 5F-hiSCs (dH1)-1and 5F-hiSCs (dH1)-2 are 5F-hiSCs (dH1), and both aSCs-1 and aSCs-2 are the adult Sertoli cells). A result shows that compared to the dH1-2K7 or the HPF-2K7, the entire down-regulated gene numbers of the 2F-hiSCs (HPF), the 5F-hiSCs (HPF), the 2F-hiSCs (dH1), the 5F-hiSCs (dH1) and the adult Sertoli cells all are 689, and the entire up-regulated gene numbers all are 512.

The result shows that the 2F-hiSCs (HPF), the 5F-hiSCs (HPF), the 2F-hiSCs (dH1), the 5F-hiSCs (dH1) and the adult Sertoli cells express similar genes, are enriched in the important biological process of the Sertoli cells, and are obviously different from the dH1-2K7 or the HPF-2K7 in expression situation.

### Step II: up-regulation of an expression level of KRT18 in the 2F-hiSCs (dH1)

The KRT18 (Gene ID: 3875) is a marker protein of the Sertoli cells, and represents the epithelization characteristic of the Sertoli cells.

The to-be-detected cells (2F-hiSCs (dH1) or dH1-2K7) are taken, and cell immunofluorescence staining is carried out. The dH1-2K7 is used as a control.

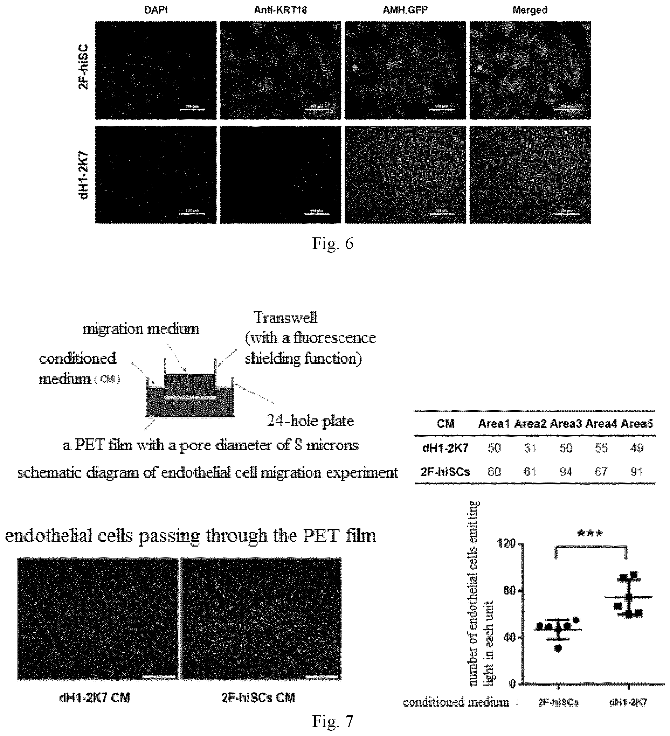

An experimental result refers to Fig. 6 (2F-hiSCs are 2F-hiSCs (dH1), cell nucleuses are marked with blue (DAPI), Anti-KRT18 is KRT18 protein immunofluorescence, AMH: GFP is fluorescence of the intracellular AMH: GFP reporter system, Merged is a staining overlay diagram, and a proportional scale is 100µm). The result shows that compared to the dH1-2K7, the expression level of KRT18 in the 2F-hiSCs (dH1) is obviously improved.

### Step III: detection on an ability that the 2F-hiSCs (dH1) attract HUVECs

1. A six-hole plate is taken, the ECM medium is added into each hole, then the six-hole plate is inoculated with the 2F-hiSCs (dH1), and an inoculation density is 50%.
2. After the step 1 is completed, the six-hole plate is taken and placed into the 5% CO₂ thermostatic incubator with the temperature of 37°C to be subjected to standing culture for 24h.
3. After the step 2 is completed, the six-hole plate is taken, the medium is discarded, then the ECM medium is added, the obtained product is placed into the 5% CO₂ thermostatic incubator with the temperature of 37°C to be subjected to standing culture for 48h, and a culture solution is collected. The culture solution is a conditioned medium for the 2F-hiSCs (dH1).
   According to the steps, the 2F-hiSCs (dH1) are replaced with the dH1-2K7, other steps are all unchanged, and a conditioned medium of the dH1-2K7 is obtained and used as a control.
4. Another six-hole plate is taken, the ECM medium is added into each hole, then the six-hole plate is inoculated with the HUVCECs, and an inoculation density is 30%.
5. After the step 4 is completed, the six-hole plate is taken and placed into the 5% CO₂ thermostatic incubator with the temperature of 37°C to be subjected to standing culture for 24h.
6. After the step 5 is completed, the six-hole plate is taken, and calcein is added to obtain a first system. In the first system, concentration of the calcein is 2.5µM.
7. After the step 6 is completed, the six-hole plate is taken and placed into the 5% CO₂ thermostatic incubator with the temperature of 37°C to be subjected to static culture for 1h, then sufficient digestion is carried out, and cells are collected.
8. After the step 7 is completed, the collected cells are taken and resuspended by the ECM medium to obtain a cell suspension with a concentration of 10⁶/mL_{∘}
9. 600µL of conditioned medium (the conditioned medium of the 2F-hiSCs (dH1) or the conditioned medium of the dH1-2K7) prepared in the step 3 is added into a substrate chamber, and then the obtained product is placed into the Transwell and infiltrated for 5min.
10. After the step 9 is completed, 100µL of cell suspension prepared in the step 8 is added into the Transwell, the obtained product is placed into the 5% CO₂ thermostatic incubator with the temperature of 37°C to be subjected to static culture for 20h, and then observation and counting are carried out under a fluorescence microscope.

An experimental result refers to Fig. 7 (the upper left is a schematic diagram of a detection device, the upper right shows migration numbers of the HUVECs of five different regions, the lower left shows an observation result under the fluorescence microscope, the lower right is a statistic result of the number of the HUVECs emitting fluorescence, 2F-hiSCs CM are the 2F-hiSCs (dH1), and dH1-2K7 CM are the dH1-2K7). The result shows that the conditioned medium for the 2F-hiSCs (dH1) have the ability of attracting more HUVECs.

### Step IV: detection on an ability that the 2F-hiSCs (dH1) form lipid droplets

BODIPY storage solution: a BODIPY dye is taken, DMSO is added to dilute to concentration of 1mg/mL, and the obtained product is preserved in a dark place at a temperature of -20°C.

APBS buffer solution is a 0.01mM PBS buffer solution with pH of 7.2.
1. A six-hole plate is taken, the MEF medium is added into each hole, then the six-hole plate is inoculated with the 2F-hiSCs (dH1), and an inoculation density is 50%.
2. After the step 1 is completed, the six-hole plate is taken and placed into the 5% CO₂ thermostatic incubator with the temperature of 37°C to be static cultured for 24h.
3. After the step 2 is completed, the six-hole plate is taken, the medium is discarded, and then the obtained product is washed once with the PBS buffer solution for 5min each time; and then a 4% (v/v) PFA aqueous solution is added, the obtained product is allowed to be static at 37°C for 20 min (for fixation).
4. After the step 3 is completed, the six-hole plate is taken, the liquid phase is discarded, a BODIPY working solution (formed by mixing 1 part by volume of BODIPY storage solution and 500 parts by volume of PBS buffer solution) is added, and staining is carried out in a dark place for 60min.
5. After the step 4 is completed, the six-hole plate is taken, the liquid phase is discarded, and the obtained product is washed for three times with the PBS buffer solution for 5min each time; and then the PBS buffer solution containing 1µg/mL of DAPI is added, and is static for 10min (for carrying out cell nucleus staining).
6. After the step 4 is completed, the six-hole plate is taken, the liquid phase is discarded, and the obtained product is washed for three times with the PBS buffer solution for 5min each time; and then the number of BODIPY positive cells is observed and counted under the fluorescence microscope.

According to the steps, the 2F-hiSCs (dH1) are replaced with the dH1-2K7, and other steps are all unchanged, and the obtained result is used as a control.

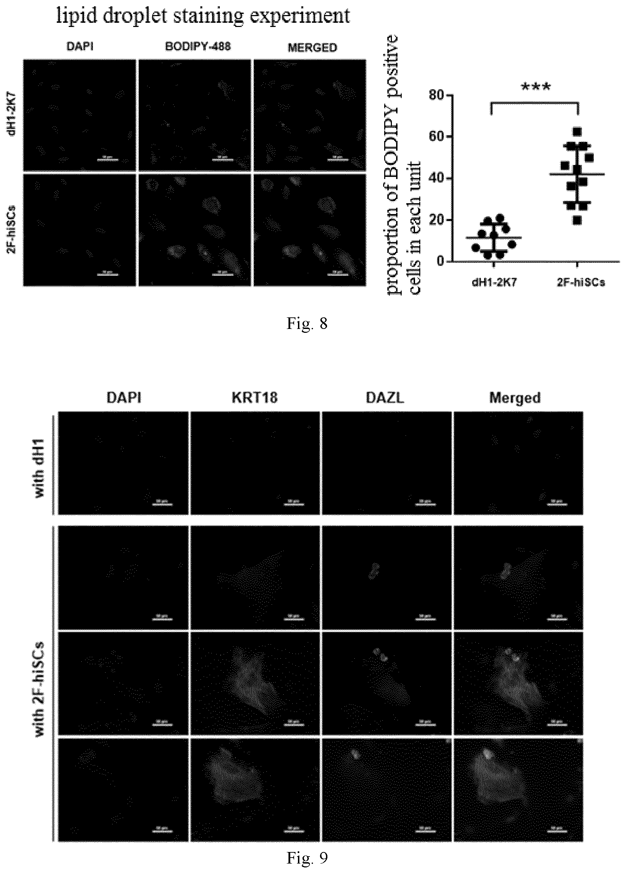

An experimental result refers to Fig. 8 (DAPI is a DAPI staining result, BODIPY-488 is a BODIPY dye staining result, Merged is staining overlay, and the 2F-hiSCs are the 2F-hiSCs (dH1)). The result shows that the 2F-hiSCs (dH1) have the ability of forming the lipid droplets.

### Step V: detection on an ability that the 2F-hiSCs (dH1) interact with germ cells

Mouse spermatogonia are derived from a C57BL/6 male mouse on the age of 6 days, and a separation method refers to the following literature: Kanatsu Shinohara M, et al. Long-term proliferation in culture and germline transmission of mouse male germline stem cells. Biology of Reproduction 69.2(2003):612.
1. A 48-hole plate is taken, and each hole is inoculated with 5×10⁴ 2F-hiSCs (dH1).
2. After the step 1 is completed, the 48-hole plate is taken, a liquid phase is discarded, the obtained product is washed for three times with the PBS buffer solution (for washing away dead falling cells).
3. After the step 2 is completed, the 48-hole plate is taken, 500µL of mouse spermatogonium suspension (which is formed by resuspending the mouse spermatogonia by the DMEMF/12 medium and has concentration of 2×10⁵/mL) is added into each hole, and the obtained product is placed into the 5% CO₂ thermostatic incubator with the temperature of 37°C to be static cultured for 48h.
4. After the step 3 is completed, the 48-hole plate is taken, and immunofluorescence identification of KRT18 (indicating the Sertoli cells) and DAZL (indicating the spermatogonia) marker proteins is carried out.

According to the steps, the 2F-hiSCs (dH1) are replaced with the dH1-2K7, other steps are all unchanged, and the obtained result is used as a control.

An experimental result refers to Fig. 9 (cell nucleuses are marked with blue (DAPI), Merged is staining overlay, with 2F-hiSCs are the 2F-hiSCs (dH1), and with dH1 is the dH1-2K7). The result shows that the 2F-hiSCs (dH1) can support attachment of the mouse germ cells better, i.e., have the ability of interacting with the germ cells.

Step VI: detection on immune privilege of the 2F-hiSCs (dH1)

### 1. Inhibition of JurKat E6-1 cell proliferation by the 2F-hiSCs (dH1)

(1) A six-hole plate is taken, the MEF medium is added into each hole, then the six-hole plate is inoculated with the 2F-hiSCs (dH1), and an inoculation density is 50%.
(2) After the step (1) is completed, the six-hole plate is taken and placed into the 5% CO₂ thermostatic incubator with the temperature of 37°C to be subjected to static culture for 24h.
(3) After the step (2) is completed, the six-hole plate is taken, the medium is discarded, then a 1640 medium containing 10% (v/v) of FBS, 1% (m/v) of glutamic acid, 1% (m/v) of non-essential amino acid and 1% (m/v) of mercaptoethanol is added, the obtained product is placed into the 5% CO₂ thermostatic incubator with the temperature of 37°C to be subjected to static culture for 48h, and a culture solution is collected. The culture solution is a conditioned medium.
(4) The conditioned medium is taken, and the 1640 medium is added to carry out dilution (a volume ratio of the conditioned medium to the 1640 medium is 4:1, 2:3, 3:2 or 1:4) so as to obtain a conditioned medium diluent.
(5) JurKat E6-1 cells are resuspended by the conditioned medium diluent to obtain a cell suspension. The JurKat E6-1 cells are resuspended by the conditioned medium to obtain a cell suspension (used as a blank control).
(6) After the step (5) is completed, a 96-hole plate is taken, the cell suspension obtained in the step (5) is added into each hole, each hole is inoculated with 2×10⁴ JurKat E6-1 cells (about 120µL), and the obtained product is placed into the 5% CO₂ thermostatic incubator with the temperature of 37°C to be subjected to static culture for 72h.
(7) After the step (6) is completed, the 96-hole plate is taken, 12µL of WST-1 solution is added into each hole, and the obtained product is placed into the 5% CO₂ thermostatic incubator with the temperature of 37°C to be subjected to static culture (in a dark place) for 3h.
(8) After the step (7) is completed, the 96-hole plate is taken, absorbance of the solution in each hole at the position of OD₄₅₀ₙₘ is read by a microplate reader, and then the relative number of the JurKat E6-1 cells in each hole is calculated. The relative number of the JurKat E6-1 cells in a certain hole=the absorbance of the solution in the hole at the position of OD₄₅₀ₙₘ/the absorbance of the blank control at the position of OD₄₅₀ₙₘ.

The relative number of the JurKat E6-1 cells in the hole where the blank control is positioned is set as 1.

According to the steps, the 2F-hiSCs (dH1) are replaced with the dH1-2K7, other steps are all unchanged, and the obtained result is used as a control.

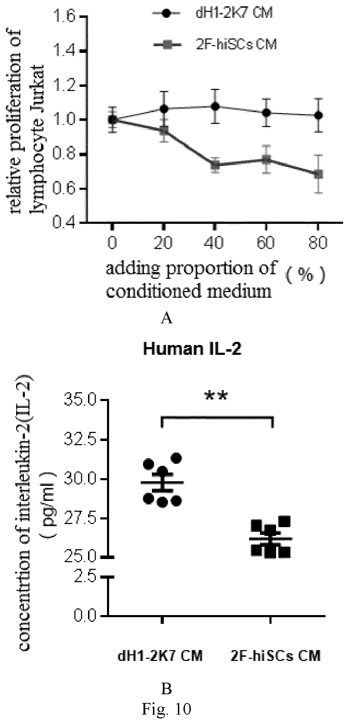

An experimental result refers to A (2F-hiSCs CM are the 2F-hiSCs (dH1), and dH1-2K7 CM are the dH1-2K7) in Fig. 10. The result shows that secreta of the 2F-hiSCs (dH1) can inhibit proliferation of the JurKat E6-1 cells.

### 2. Inhibition from production of interleukin IL-2 of JurKat cells

(1) A six-hole plate is taken, the MEF medium is added into each hole, then the six-hole plate is inoculated with 2F-hiSCs (dH1), and an inoculation density is 50%.
(2) After the step (1) is completed, the six-hole plate is taken and placed into the 5% CO₂ thermostatic incubator with the temperature of 37°C to be subjected to static culture for 24h.
(3) After the step (2) is completed, the six-hole plate is taken, the medium is discarded, then the 1640 medium containing 10% (v/v) of FBS, 1% (m/v) of glutamic acid, 1% (m/v) of non-essential amino acid and 1% (m/v) of mercaptoethanol is added, the obtained product is placed into the 5% CO₂ thermostatic incubator with the temperature of 37°C to be subjected to static culture for 48h, and a culture solution is collected. The culture solution is a conditioned medium.
(4) One part by volume of conditioned medium and one part by volume of 1640 medium are mixed to obtain a diluent. The JurKat E6-1 cells are resuspended by the diluent to obtain a cell suspension.
(5) After the step (4) is completed, the six-hole plate is taken, the cell suspension obtained in the step (4) is added into each hole, each hole is inoculated with 2×10⁵ JurKat E6-1 cells (about 2mL), and the obtained product is placed into the 5% CO₂ thermostatic incubator with the temperature of 37°C to be subjected to static culture for 72h.
(6) After the step (5) is completed, centrifugation is carried out, precipitates are collected, then 200µL of RIPA solution is added to carry out pyrolysis so as to obtain a lysate. IL-2 concentration in the lysate is detected by adopting the ELASA kit.

According to the steps, the 2F-hiSCs (dH1) are replaced with the dH1-2K7, other steps are all unchanged, and the obtained result is used as a control.

An experimental result refers to B (2F-hiSCs CM are the 2F-hiSCs (dH1), and dH1-2K7 CM are the dH1-2K7) in Fig. 10. The result shows that the secreta of the 2F-hiSCs (dH1) can inhibit production of the interleukin IL-2.

### 3. In-vivo cotransplantation experiment of human 293FT cells in mice

(1) The 293FT cells containing a luciferase reporter system is constructed.
   a. A luciferase gene (the genebank number is: MF693179.1) is inserted to a recognition site of the restriction endonuclease EcoRI of the pENTR/1A plasmid so as to obtain a vector pENTR/1A-luc.
   b. Double-stranded DNA molecules (a nucleotide sequence of an EF1α promoter) as shown in the SEQ ID No. 2 in the sequence list are inserted to the recognition site of the restriction endonuclease EcoRI of the pENTR/5' topo plasmid so as to obtain a vector pENTR/5' topo-EF1α.
   c. The vector pENTR/1A-luc, the vector pENTR/5' topo-EF1α and the lentivirus plasmid p2K7-NEO are taken to prepare a lentivirus vector by homologous recombination.
   d. The lentivirus vector prepared in the step c, the helper plasmid vsvg and the lentivirus vector packaging plasmid pCMV ΔR8.9 are co-transfected with 293FT cells to obtain the 293FT cells containing the luciferase reporter system.
(2) 100µL of Matrigel stock solution, about 1.3×10⁶ 293FT cells containing the luciferase reporter system and about 2.5×10⁵ 2F-hiSCs (dH1) are mixed to obtain mixed liquid 1. 100µL of Matrigel stock solution, about 1.3×10⁶ 293FT cells containing the luciferase reporter system and about 2.5×10⁵ dH1-2K7 are mixed to obtain mixed liquid 2. Both the mixed liquid 1 and the mixed liquid 2 are placed on ice to be taken into an animal operation platform.
(3) Three healthy C57B6/6J mice are taken and are anesthetized by intraperitoneal injection of 1.2% (m/v) of Avertin solution, and an injection dosage is 0.2mL per 10g of weight.
(4) After the step (3) is completed, the mixed liquid 1 is subcutaneously injected to armpits of left fore limbs of the mice, the mixed liquid 2 is subcutaneously injected to armpits of right fore limbs of the mice, after the Matrigel stock solution is solidified, the C57B6/6J mice are place back to a culture cage, and then survival conditions of 293FT at the mouse transplantation positions (i.e., the armpits of the fore limbs of the mice) are monitored by utilizing an in-vivo imager. The operation of in-vivo imaging is as follows: 15min before monitoring, a luciferase substrate aqueous solution with a concentration of 15mg/mL is intraperitoneally injected, an injection dosage is 10µL per 1g of weight; and the mice are placed in the in-vivo imager to detect strength of bioluminescence.

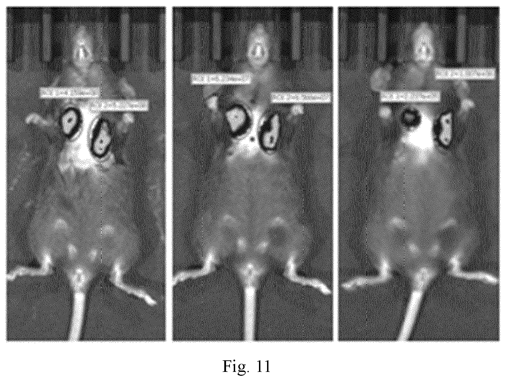

An experimental result refers to Fig. 11 (from left to right, they sequentially are the mice on the first day, the fourth day and the sixth day of injection). The result shows that the 2F-hiSCs (dH1) can prolong the allogeneic survival ability of the 293FT cells, and has an immune privilege function.

According to the steps II to VI, the 2F-hiSCs (dH1) are replaced with the 5F-hiSCs (dH1), the 2F-hiSCs (HPF) or the 5F-hiSCs (HPF), and other steps are all unchanged. A result shows that compared to the 2F-hiSCs (dH1), the 5F-hiSCs (dH1), the 2F-hiSCs (HPF) and the 5F-hiSCs (HPF) have no obvious difference in result.

According to the steps, the 2F-hiSCs (dH1) are replaced with other hiSCs (dH1) (i.e., the protein combination expressed by the recombinant lentivirus in the virus fluid in the step 2 of the step I in Embodiment 2 is No.3, No.7, No.10, No.14, No.16, No.18, No.19, No.21, No.22, No.25, No.26, No.27, No.29 or No.30 in Table 1) and other hiSCs (HPF) (i.e., the protein combination expressed by the recombinant lentivirus in the virus fluid in the step II in Embodiment 2 is No.3, No.7, No.10, No.14, No.16, No.18, No.19, No.21, No.22, No.25, No.26, No.27, No.29 or No.30), and other steps are all unchanged. A result shows that compared to the 2F-hiSCs (dH1), other hiSCs (dH1) and other hiSCs (HPF) have no obvious difference in result.

From the above, the hiSCs (dH1) and the hiSCs (HPF) prepared in Embodiment 2 highly express the KRT18 and present an epithelial appearance, their genes are enriched in the important biological process of the Sertoli cells, and the hiSCs (dH1) and the hiSCs (HPF) have the characteristics of attracting the endothelial cells, forming the lipid droplets, interacting with the germ cells, inhibiting lymphocyte growth and interleukin production, having immune privilege and the like, and fully acquire the characteristics of the Sertoli cells. Thus it can be seen that both the hiSCs (dH1) and the hiSCs (HPF) prepared in Embodiment 2 are the Sertoli cells.

### Embodiment 4: Influence of AMH:GFP Reporter System on acquisition of Sertoli Cells

### Step I: construction of an AMH-D:GFP reporter system

1, The inventor of the present disclosure clones a second promoter of the AMH gene from the human genome DNA. A length of the second promoter of the AMH gene is about 726bp, and a nucleotide sequence of the second promoter is as shown in a SEQ ID No. 3 in the sequence list.
2. After the step 1 is completed, an encoding gene (i.e., an eGFP gene) of a fluorescent protein eGFP is connected to a 3' terminal of the second promoter of the AMH gene so as to obtain the AMH-D:GFP reporter system. In the AMH-D:GFP reporter system, the second promoter of the AMH gene drives expression of the fluorescent protein eGFP.

### Step II: construction of a recombinant lentivirus containing the AMH-D:GFP reporter system

1. The eGFP gene is inserted to the recognition site of the restriction endonuclease EcoRI of the pENTR/1A plasmid so as to obtain the vector pENTR/1A-eGFP.
2. Double-stranded DNA molecules as shown in the SEQ ID No. 3 in the sequence list are inserted to the recognition site of the restriction endonuclease EcoRI of the pENTR/5' topo plasmid so as to obtain a vector pENTR/5' topo-AMH-D.
3. The vector pENTR/1A-eGFP, the vector pENTR/5' topo-AMH-D and the lentivirus plasmid p2K7-NEO are taken to prepare a lentivirus vector by homologous recombination.
4. The lentivirus vector prepared in the step 3, the helper plasmid vsvg and the lentivirus vector packaging plasmid pCMV ΔR8.9 are co-transfected with the 293FT cells to obtain the recombinant lentivirus containing the AMH-D:GFP reporter system.

Step III: according to the step III in Embodiment 1 and the steps in Embodiment 2 and the steps in Embodiment 3, replacement of the AMH:GFP reporter system in the step III in Embodiment 1 with the AMH-D:GFP reporter system without changing other steps.

Part of results are as follows: when the AMH-D:GFP reporter system is adopted, in the system 2 (the NR5A1 protein and the GATA4 protein expressed by the recombinant lentivirus in the virus fluid) subjected to the step 8 in the step I in Embodiment 2, 1.23% of cells are AMH-D:GFP+Sertoli cells; and in the system 2 (the NR5A1 protein, the GATA4 protein, the WT1 protein, the SOX9 protein and the DMRT1 protein expressed by the recombinant lentivirus in the virus fluid) subjected to the step 8 in the step II in Embodiment 2, 0.86% of cells are AMH-D:GFP+Sertoli cells. Those AMH-D:GFP+Sertoli cells highly express the KRT18 and present the epithelial appearance, their genes are enriched in the important biological process of the Sertoli cells, and the AMH-D:GFP+Sertoli cells have the characteristics of attracting the endothelial cells, forming the lipid droplets, interacting with the germ cells, inhibiting lymphocyte growth and interleukin production, having immune privilege and the like, and fully acquire the characteristics of the Sertoli cells. Thus, it can be seen that the Sertoli cells can also be obtained by adopting the AMH-D:GFP reporter system, but a proportion of the obtained Sertoli cells is obviously reduced.

### Industrial Application

The Sertoli cells prepared by adopting the method provided by the present disclosure provides a rich cell source for constructing a complete in-vitro sperm differentiation system and researching an interaction mechanism between the Sertoli cells and male germ cells. The present disclosure has important application value in the fields of infertility treatment, allogeneic transplantation, cell therapy and the like.

## Claims

1. A method for preparing Sertoli cells, comprising a step a2): increasing the expression level and/or activity of a protein combination in fibroblasts,
wherein the protein combination is selected from one of the following groups:
B2) NR5A1 and GATA4,
B6) NR5A1, GATA4 and WT1,
B7) NR5A1, GATA4 and SOX9,
B8) NR5A1, GATA4 and DMRT1,
B12) NR5A1, GATA4, WT1 and SOX9,
B13) NR5A1, GATA4, WT1 and DMRT1,
B14) NR5A1, GATA4, SOX9 and DMRT1.

2. The method according to claim 1, wherein the method further comprises a step a1):
introducing a fluorescent protein reporter system specifically expressed in the Sertoli cells into the fibroblasts; wherein the fluorescent protein reporter system specifically expressed in the Sertoli cells, a promoter specifically expressed in the Sertoli cells promotes expression of a fluorescent protein; and
the step a1) is carried out before the step a2) is carried out,
wherein the promoter specifically expressed in the Sertoli cells is a promoter of an AMH gene.

3. The method according to claim 2, wherein the nucleotide sequence of the promoter of the AMH gene is as shown SEQ ID No. 1 in the sequence list.

4. The method according to claim 2, wherein the fluorescent protein is a green fluorescent protein, a yellow fluorescent protein or a red fluorescent protein.

5. The method according to any one of claims 1 to 4, further comprising steps a3) to a6):
a3): after completing the step a2), adding G418 into a culture system of the fibroblasts to obtain a medicinal sieve system;
a4): after completing the step a3), taking the medicinal sieve system and culturing for 3-7 d;
a5): after completing the step a4), discarding the liquid phase, adding a medium for culturing fibroblasts and continuing culturing for 3-7 d; and
a6): after completing the step a5), isolating the Sertoli cells from the culture system.

6. The method according to claim 5, wherein in the step a6), isolating the Sertoli cells is implemented by isolating cells capable of emitting fluorescence.

7. Use of a protein combination for preparing Sertoli cells from fibroblasts, wherein the protein combination is selected from one of the following groups:
B2) NR5A1 and GATA4,
B6) NR5A1, GATA4 and WT1,
B7) NR5A1, GATA4 and SOX9,
B8) NR5A1, GATA4 and DMRT1,
B12) NR5A1, GATA4, WT1 and SOX9,
B13) NR5A1, GATA4, WT1 and DMRT1,
B14) NR5A1, GATA4, SOX9 and DMRT1.

8. The use according to claim 7, the protein combination and a fluorescent protein reporter system in fibroblasts for preparing Sertoli cells,
wherein the fluorescent protein reporter system specifically expressed in the Sertoli cells, a promoter specifically expressed in the Sertoli cells promotes expression of a fluorescent protein, wherein the promoter specifically expressed in the Sertoli cells is a promoter of an AMH gene.

## Patentansprüche

1. Ein Verfahren zum Vorbereiten von Sertolizellen, das einen Schritt a2) beinhaltet:
Erhöhen des Expressionsspiegels und/oder der Aktivität einer Proteinkombination in Fibroblasten,
wobei die Proteinkombination aus einer der folgenden Gruppen ausgewählt ist:
B2) NR5A1 und GATA4,
B6) NR5A1, GATA4 und WT1,
B7) NR5A1, GATA4 und SOX9,
B8) NR5A1, GATA4 und DMRT1,
B12) NR5A1, GATA4, WT1 und SOX9,
B13) NR5A1, GATA4, WT1 und DMRT1,
B14) NR5A1, GATA4, SOX9 und DMRT1.

2. Verfahren gemäß Anspruch 1, wobei das Verfahren ferner einen Schritt a1) beinhaltet:
Einführen eines Fluoreszenzproteinreportersystems, das in den Sertolizellen spezifisch exprimiert wird, in die Fibroblasten; wo in dem Fluoreszenzproteinreportersystem, das in den Sertolizellen spezifisch exprimiert wird, ein Promotor, der in den Sertolizellen spezifisch exprimiert wird, die Expression eines Fluoreszenzproteins fördert; und
Schritt a1) ausgeführt wird, bevor Schritt a2) ausgeführt wird,
wobei der Promotor, der in den Sertolizellen spezifisch exprimiert wird, ein Promotor eines AMH-Gens ist.

3. Verfahren gemäß Anspruch 2, wobei die Nukleotidsequenz des Promotors des AMH-Gens wie in SEQ ID NO: 1 in der Sequenzliste gezeigt ist.

4. Verfahren gemäß Anspruch 2, wobei das Fluoreszenzprotein ein grünes Fluoreszenzprotein, ein gelbes Fluoreszenzprotein oder ein rotes Fluoreszenzprotein ist.

5. Verfahren gemäß einem der Ansprüche 1 bis 4, das ferner die Schritte a3) bis a6) beinhaltet:
a3): nach dem Beenden von Schritt a2), Hinzufügen von G418 zu einem Kultursystem der Fibroblasten, um ein medizinisches Siebsystem zu erhalten;
a4): nach dem Beenden von Schritt a3), Heranziehen des medizinisichen Siebsystems und Züchten von 3-7 d;
a5): nach dem Beenden von Schritt a4), Ableiten der Flüssigphase, Hinzufügen eines Mediums zum Züchten von Fibrobasten und Weiterführens des Züchtens von 3-7 d;
und
a6): nach dem Beenden des Schritts a5), Isolieren der Sertolizellen von dem Kultursystem.

6. Verfahren gemäß Anspruch 5, wobei in Schritt a6) das Isolieren der Sertolizellen durch Isolieren von Zellen, die zum Emittieren von Fluoreszenz fähig sind, implementiert wird.

7. Verwenden einer Proteinkombination zum Vorbereiten von Sertolizellen aus Fibroblasten, wobei die Proteinkombination aus einer der folgenden Gruppen ausgewählt ist:
B2) NR5A1 und GATA4,
B6) NR5A1, GATA4 und WT1,
B7) NR5A1, GATA4 und SOX9,
B8) NR5A1, GATA4 und DMRT1,
B12) NR5A1, GATA4, WT1 und SOX9,
B13) NR5A1, GATA4, WT1 und DMRT1,
B14) NR5A1, GATA4, SOX9 und DMRT1.

8. Verwendung gemäß Anspruch 7, die Proteinkombination und ein Fluoreszenzproteinreportersystem in Fibroblasten zum Vorbereiten von Sertolizellen, wo in dem Fluoreszenzproteinreportersystem, das in den Sertolizellen spezifisch exprimiert wird, ein Promotor, der in den Sertolizellen spezifisch exprimiert wird, die Expression eines Fluoreszenzproteins fördert, wobei der Promotor, der in den Sertolizellen spezifisch exprimiert wird, ein Promotor eines AMH-Gens ist.

## Revendications

1. Un procédé pour la préparation de cellules de Sertoli, comprenant une étape a2) :
augmenter le niveau d'expression et/ou l'activité d'une combinaison de protéines dans des fibroblastes,
où la combinaison de protéines est sélectionnée dans l'un des groupes suivants :
B2) NR5A1 et GATA4,
B6) NR5A1, GATA4 et WT1,
B7) NR5A1, GATA4 et SOX9,
B8) NR5A1, GATA4 et DMRT1,
B12) NR5A1, GATA4, WT1 et SOX9,
B13) NR5A1, GATA4, WT1 et DMRT1,
B14) NR5A1, GATA4, SOX9 et DMRT1.

2. Le procédé selon la revendication 1, le procédé comprenant en sus une étape a1) :
introduire dans les fibroblastes un système rapporteur de protéine fluorescente spécifiquement exprimé dans les cellules de Sertoli ; où le système rapporteur de protéine fluorescente spécifiquement exprimé dans les cellules de Sertoli, un promoteur spécifiquement exprimé dans les cellules de Sertoli promeut l'expression d'une protéine fluorescente ; et
l'étape a1) est effectuée avant que l'étape a2) soit effectuée,
où le promoteur spécifiquement exprimé dans les cellules de Sertoli est un promoteur d'un gène RAM.

3. Le procédé selon la revendication 2, où la séquence nucléotidique du promoteur du gène RAM est telle que représentée dans SEQ ID NO : 1 dans la liste de séquences.

4. Le procédé selon la revendication 2, où la protéine fluorescente est une protéine fluorescente verte, une protéine fluorescente jaune ou une protéine fluorescente rouge.

5. Le procédé selon l'une quelconque des revendications 1 à 4, comprenant en sus les étapes a3) à a6) :
a3) : après avoir terminé l'étape a2), ajouter de la G418 dans un système de culture des fibroblastes afin d'obtenir un système de tamisage médicinal ;
a4) : après avoir terminé l'étape a3), prendre le système de tamisage médicinal et cultiver pendant 3 à 7 j ;
a5) : après avoir terminé l'étape a4), jeter la phase liquide, ajouter un milieu pour cultiver des fibroblastes et continuer la culture pendant 3 à 7 j ; et
a6) : après avoir terminé l'étape a5), isoler les cellules de Sertoli du système de culture.

6. Le procédé selon la revendication 5, où, dans l'étape a6), le fait d'isoler les cellules de Sertoli est mis en œuvre par isolement de cellules capables d'émettre une fluorescence.

7. Utilisation d'une combinaison de protéines pour la préparation de cellules de Sertoli à partir de fibroblastes, où la combinaison de protéines est sélectionnée dans l'un des groupes suivants :
B2) NR5A1 et GATA4,
B6) NR5A1, GATA4 et WT1,
B7) NR5A1, GATA4 et SOX9,
B8) NR5A1, GATA4 et DMRT1,
B12) NR5A1, GATA4, WT1 et SOX9,
B13) NR5A1, GATA4, WT1 et DMRT1,
B14) NR5A1, GATA4, SOX9 et DMRT1.

8. L'utilisation selon la revendication 7, la combinaison de protéines et un système rapporteur de protéine fluorescente dans des fibroblastes pour la préparation de cellules de Sertoli,
où le système rapporteur de protéine fluorescente spécifiquement exprimé dans les cellules de Sertoli, un promoteur spécifiquement exprimé dans les cellules de Sertoli promeut l'expression d'une protéine fluorescente, où le promoteur spécifiquement exprimé dans les cellules de Sertoli est un promoteur d'un gène RAM.
